# EUROPEAN PATENT APPLICATION

(11) **EP 4 442 250 A1**
(43) Date of publication of application: **09.10.2024**
(21) Application number: 24020103.8
(22) Date of filing: 03.04.2024
(51) Int. Cl.: A61K 9/00, A23L 33/00, A61K 9/16, A61K 9/50

(54) **PHARMACEUTICAL PRODUCT AND RELATED PREPARATION METHOD**

(30) Priority: 03.04.2023 IT 202300006492
(71) Applicant: Linea Blu S.r.l., 47892 Acquaviva (SM) (SM)
(72) Inventor: Sanchini, Silvano, 47893 BORGO MAGGIORE (SM) (SM)
(74) Representative: Montebelli, Marco

(57) **Abstract**

An orally soluble pharmaceutical product comprises a core (1) comprising a gummy candy (11), a coating (2) and active ingredients (3), wherein the active ingredients (3) are contained in the coating (2).

## Description

The invention concerns a pharmaceutical product, in particular exploitable in the form of a food supplement, and its preparation method.

The definition of a pharmaceutical product refers to those products, whether medicinal or not, capable of producing functional changes in a living organism through a chemical-physical action: therefore, cosmetic products fall into this category, in addition to medicines, herbal and food supplements.

Many pharmaceutical products, in particular medicines and supplements, are made in solid forms and intended to be taken orally.

Although powders are always at the base of the preparation methods, the finished product can also be presented in the form of capsules or tablets, depending on the type of function it will perform.

While medicines and powder supplements do not require special processing and are usually packed in sachets, capsules and tablets require a more articulate and complex treatment that often ends with the application of a coating that encloses a central core in which are at least part of the active ingredients of the pharmaceutical product.

This coating can be applied in various ways: among the most common are the dry coating, the pelleting and application of a film.

The dry coating is realized thanks to special compression machines that house the core of the product in a compression chamber and arrange to cover it with a granular material distributed around the core and pressed by one or two compressions.

Pelleting is the oldest method of coating, originally used to produce "confetti" (candies). The cores to be coated are moved, inside a machine called coating pan, and wetted with an aqueous solution containing sucrose and other substances; the introduction of hot air inside the coating pan promotes the evaporation of the solution leaving a hard, thick and resistant coating on the core.

The application of a film has gradually replaced pelleting in recent decades: it consists in coating the core with a thin polymer membrane. More precisely, nebulized drops of a polymer solution or polymer dispersion are sprayed onto the moving cores within the coating pan, adhering to them and coalesc with nearby drops to form a thin film from which the solvent evaporates rapidly.

Depending on whether the core coating is "soft" or "hard", the coating application process is classified as "soft panning" or "hard panning".

Today there are coating pans of numerous geometric shapes, whose interior is heated in different ways; regardless of the method used, this need, however, precludes the use of certain active ingredients that cannot be treated at temperatures reached within the coating pan, normally between 50 and 60 °C.

The purpose of this invention is therefore to eliminate the inconveniences aforementioned.

The invention, as characterised by claims, achieves the purpose by concentrating the active ingredients in a cold-applied coating.

The main advantage obtained through this invention lies essentially in the fact that the cold preparation of the coating allows the use of any active substance.

In addition, the method of preparation is more economical as it does not have to use energy sources to heat the coating pan.

Further advantages and characteristics of the invention will be more evident in the following detailed description, made with reference to the drawings, which represent a non-exhaustive example of execution, wherein:
- Figure 1 shows the invention according to a partially sectioned view;
- Figure 2 shows a step of the preparation method.

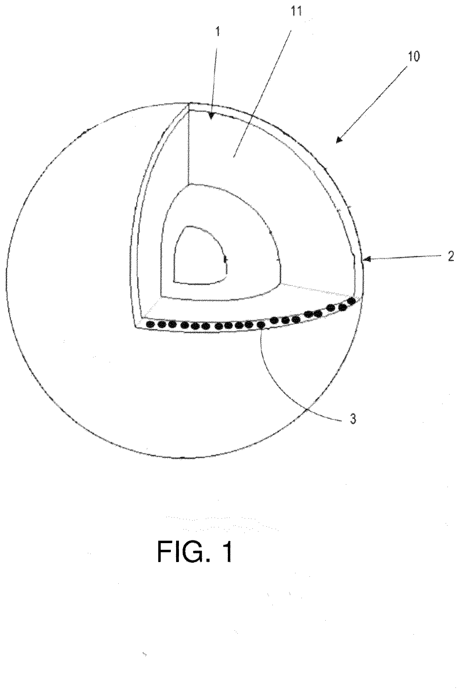

The invention concerns an orally soluble pharmaceutical product, schematically represented in Figure 1, comprising a core (1), a coating (2) and active ingredients (3), wherein the coating (2) contains all the active ingredients (3) of the pharmaceutical product (10).

The core (1) can take several forms: in a preferred solution it consists of a gummy candy (11), or gelée.

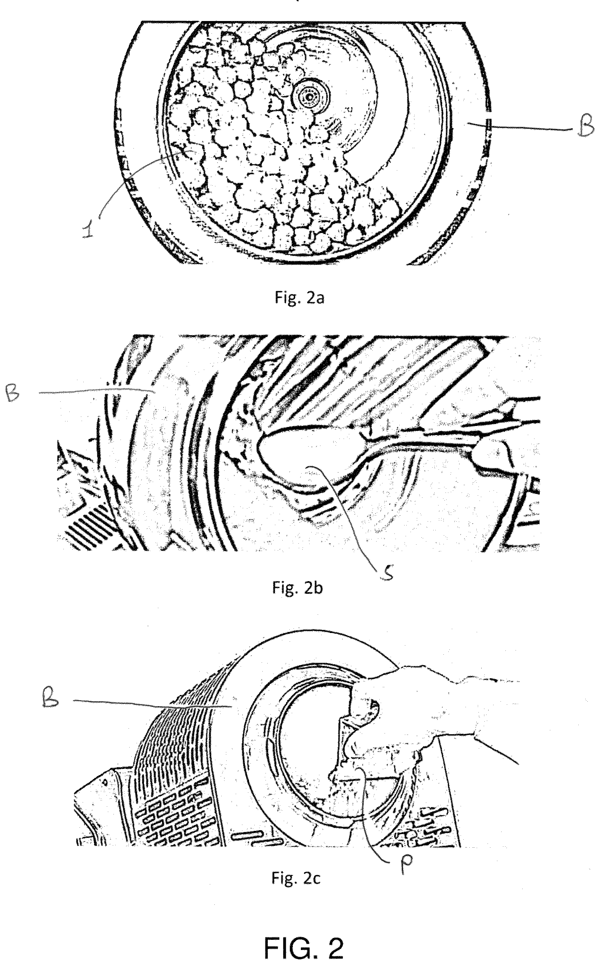

When the soft candies are processed in a coating pan (B), visible in Figure 2a, they tend to easily deform: for this reason it is advisable that at each processing not more than 30 kilograms of cores (1) are placed in the coating pan (B), unlike what usually happens, with quantities that can also exceed the quintal. Alternatively, it may be convenient for the gummy candy (11) to be pre-frozen, so that larger quantities of cores can be processed simultaneously (1).

A method of preparing a pharmaceutical product (10) of the type described above is partially represented in Figure 2. It comprises at least a first step of preparing of an edible core (1): as already mentioned, it can be made up, for example, by a soft candy, or gelée, which may also have been pre-frozen.

Then, separately, a water-based solution (S), essentially a neutral syrup, containing at least one sweetener in an amount by weight of between 50% and 80% of the total weight of the solution (S) and a powder (P) containing at least one sweetener in an amount by weight of at least 20% of the total weight of the powder (P) are prepared.

The term sweetener is intended to refer to any substance suitable for sweetening a product intended for oral use: it may therefore consist of natural sugars, such as sucrose, fructose and lactose, other natural substances, such as erythritol, honey and stevia, or synthetic sweeteners, such as saccharin and aspartame.

The sweetener is present in the solution (S) with a variable concentration normally measured in Brix degrees (°Bx), defined as the percentage by weight of the solid dissolved in the liquid with respect to the total weight of the solution (S). In this case, an optimal concentration can be between 60 and 70 °Bx.

At this point, at least one active ingredient (3) is added to the solution (S) and/or to the powder (P): when the active ingredient (3) is added to the powder (P), it can reach a percentage by weight up to 80% of the total weight of the powder (P).

A preferred composition however provides that the powder (P) comprises a percentage by weight of active ingredients (3) ranging between 50% and 70% and a percentage by weight of sweetener ranging between 30% and 50%. In some cases, the active ingredient (3) or a set of these is also, or only, added to the solution (S), in variable quantities according to numerous factors.

Unlike the known procedures, the application of the coating (2) is carried out inside a coating pan (B) at room temperature, at a temperature not exceeding 20 °C: after the cores (1) are moved, as it can be seen in figure 2a, the solution (S) is poured alternately with the powder (P), in order to obtain a coating (2) with alternating layers of solution (S) and powder (P), as seen in Figures 2b and 2c.

If a pre-freezing step of the cores (1) is carried out, it is preferable that during the movement of the cores (1) inside the coating pan (B) liquid nitrogen is occasionally introduced to maintain the temperature sufficiently low, approximately around 0 °C.

To obtain a good result, it is preferable that the quantities of solution (S) and powder (P) are alternately spread over the cores (1) in a proportion by weight ranging between 1 to 2 and 1 to 7; that is, if 100 grams of solution (S) are poured in the next step it is preferable to pour between 200 and 700 grams of powder (P), continuing until the formation of a coating (2) that can vary in thickness from less than 1 millimeter up to about 4/5 millimeters.

The pharmaceutical product (10), in particular a food supplement, so obtained from the gummy candies (11) is removed from the coating pan (B) and left to stand for drying for a time preferably ranging between 12 and 24 hours.

Then, the pharmaceutical product (10) can be subjected to a further freezing step, and finally a lacquering and polishing step is performed, after which the pharmaceutical product (10) is left to rest for another 12/24 hours before being packaged.

## Claims

1. Orally soluble pharmaceutical product, comprising a core (1), a coating (2) and active ingredients (3), **characterised in that** the coating (2) contains all active ingredients (3) of the pharmaceutical product (10) and **in that** the core (1) comprises a gummy candy (11).

2. Pharmaceutical product according to claim 1, **characterised in that** the gummy candy (11) is pre-frozen.

3. Method of preparing a pharmaceutical product, **characterised by** comprising at least the following steps:
- preparing an edible core (1) comprising a gummy candy (11);
- preparing a water based solution (S) containing at least one sweetener in an amount by weight ranging between 50% and 80% of the total weight of the solution (S);
- preparing a powder (P), containing at least one sweetener in an amount by weight at least equal to 20% of the total weight of the powder (P);
- adding at least one active ingredient (3) to the solution (S) and/or to the powder (P);
- moving the cores (1) in a coating pan (B) at room temperature;
- spreading the solution (S) and the powder (P) in the coating pan (B) at alternating paces, as to make a coating (2) having solution (S) and powder (P) alternating layers;
- extracting the pharmaceutical product (10) thus obtained from the coating pan (B).

4. Method according to claim 3, **characterised in that** the solution (S) and the powder (P) are alternately spread according to a weight ratio ranging between 1 to 2 and 1 to 7.

5. Method according to claim 3 or 4, **characterised in that** the solution (S) contains at least one sweetener in an amount by weight ranging between 60% and 70% of the total weight of the solution (S).

6. Method according to claim 3 or 4 or 5, **characterised in that** the powder (P) contains at least one sweetener in an amount by weight ranging between 20% and 60% of the total weight of the powder (P).

7. Method according to any one of the claims from 3 to 6, **characterised by** comprising a preliminary step of pre-freezing of the cores (1) comprising a gummy candy (11).

8. Method according to any one of the claims from 3 to 7, **characterised by** comprising, after the step of extracting of the pharmaceutical product (10) from the coating pan (B), a drying phase for a time ranging between 12 and 24 hours.

9. Method according to any one of the claims from 3 to 8, **characterised by** comprising a freezing phase of the pharmaceutical product (10), following the drying phase.

10. Method according to any one of the claims from 3 to 9, **characterised by** comprising a final step of lacquering and polishing, after which the pharmaceutical product (10) is made to rest for another 12/24 hours before being packaged.

11. Method according to claim 7, **characterised in that** during the step of moving the cores (1) in the coating pan (B), liquid nitrogen is injected to reduce the temperature.
